# EUROPEAN PATENT SPECIFICATION

(11) **EP 3 449 882 B1**
(45) Date of publication and mention of the grant of the patent: **30.09.2020**
(21) Application number: 18190308.9
(22) Date of filing: 22.08.2018
(51) Int. Cl.: A61F 13/00, A61B 5/01, A61B 5/053, A61F 7/00

(54) **SYSTEMS FOR MONITORING WOUNDS AND WOUND DRESSING STATUS**
SYSTEME ZUR ÜBERWACHUNG VON WUNDEN UND DES WUNDVERBANDSTATUS
SYSTÈMES DE SURVEILLANCE D'ÉTAT DE PLAIES ET DE PANSEMENTS

(30) Priority: 30.08.2017 US 201762551861 P; 27.07.2018 US 201816047412
(43) Date of publication of application: 06.03.2019
(73) Proprietor: Hill-Rom Services, Inc., Batesville, IN 47006-9167 (US)
(72) Inventor: RIBBLE, David, Batesville, Indiana 47006-9167 (US); FU, Yongji, Batesville, Indiana 47006-9167 (US); EMMONS, Kirsten, Batesville, Indiana 47006-9167 (US); LACHENBRUCH, Charles, Batesville, Indiana 47006-9167 (US); LAWRENCE, Bob, Batesville, Indiana 47006-9167 (US); HOOD, Michael, Batesville, Indiana 47006-9167 (US); MYERSON, Craig, Batesville, Indiana 47006-9167 (US)
(74) Representative: Findlay, Alice Rosemary

(56) References cited:
- US-A1- 2003 167 029
- US-A1- 2015 264 452
- US-A1- 2015 265 191
- US-A1- 2016 015 962
- US-A1- 2016 228 049
- US-A1- 2017 065 751

## Description

The present specification generally relates to systems for monitoring wounds and the status of wound dressings and system for protecting wounds, more particularly, to systems for monitoring wounds and the status of wound dressings based on data measured by one or more sensing devices attached to the wound dressings, and to systems for protecting wounds.

Wounds are covered by wound dressings in order to prevent the wounds from being infected. Health care providers frequently and sometimes unnecessarily change the dressings, which exposes the wounds to environment, and incurs heat loss of the wounds. In addition, the status of wounds may not be checked unless wound dressings are removed.

US2015/264452A1 describes computational systems and methods for reporting information regarding appurtenances to wound dressings. A system can include an appurtenance to a wound dressing, including a substrate, a transmission unit, a selectively activated switch, and a projection of a size and shape to extend into an interior region of a wound dressing and configured to sample a fluid associated with a wound; a local unit, including a receiver configured to receive signals from the transmission unit, a transmitter configured to send signals to the transmission unit, a processor, non-volatile memory, and a power source; and a central assembly, including a processor, a receiver configured to receive signals from the local unit, and at least one user interface.

US-2015/265191-A1 shows a wound dressing for detecting infections by monitoring a decrease in pH.

Accordingly, it may be beneficial to provide systems for monitoring wounds and the status of wound dressings based on data measured by one or more sensing devices attached to the wound dressings.

The present invention is defined by the claims.

In an embodiment, a system for monitoring a wound includes a wound dressing configured to cover a wound surface of the wound, one or more sensing devices attached to the wound dressing, the one or more sensing devices comprising a pH meter, a network interface hardware, and a controller. The controller includes one or more processors, and one or more memory modules storing computer readable and executable instructions. The controller receives data measured by the one or more sensing devices along with times of measuring, determines whether a level of pH measured by the pH meter is increasing over time based on the data and times of measuring, determines that an infection is likely to occur when the level of the pH measured by the pH meter increases over time, and outputs an alert, through the network interface hardware, in response to determining that the infection is likely to occur.

The invention will now be further described by way of example with reference to the accompanying drawings, in which:
FIG. 1 schematically depicts a wound management system, according to one or more embodiments shown and described herein;
FIG. 2 schematically depicts a wound management system including a power element according to one or more embodiments shown and described herein;
FIGS. 3A and 3B depict a wound management system including one or more sensing devices according to one or more embodiments shown and described herein;
FIG. 4 schematically depicts a wound management system including one or more sensing devices, according to one or more embodiments shown and described herein;
FIG. 5A depicts a flowchart for determining whether an infection is likely to occur, according to one or more embodiments shown and described herein;
FIG. 5B depicts a flowchart for determining whether a dressing needs to be changed, according to one or more embodiments shown and described herein;
FIGS. 6A and 6B depict a wound protection system having a knob configured to create a vacuum seal, according to one or more embodiments shown and described herein; and
FIG. 6C depicts a top view of the wound protection system having a knob configured to create a vacuum seal, according to one or more embodiments shown and described herein.

Reference will now be made in detail to embodiments of wound monitoring systems and wound protection systems, examples of which are illustrated in the accompanying drawings. Whenever possible, the same reference numerals will be used throughout the drawings to refer to the same or like parts. In one embodiment, a wound monitoring system includes a wound dressing configured to cover a wound surface of the wound, one or more sensing devices attached to the wound dressing, a network interface hardware, and a controller. The controller includes one or more processors, and one or more memory modules storing computer readable and executable instructions. The controller receives data measured by the one or more sensing devices, determines whether an infection is likely to occur based on the data measured by the one or more sensing devices, and outputs an alert, through the network interface hardware, in response to determining that the infection is likely to occur. Various embodiments of wound management systems will be described herein with specific reference to the appended drawings.

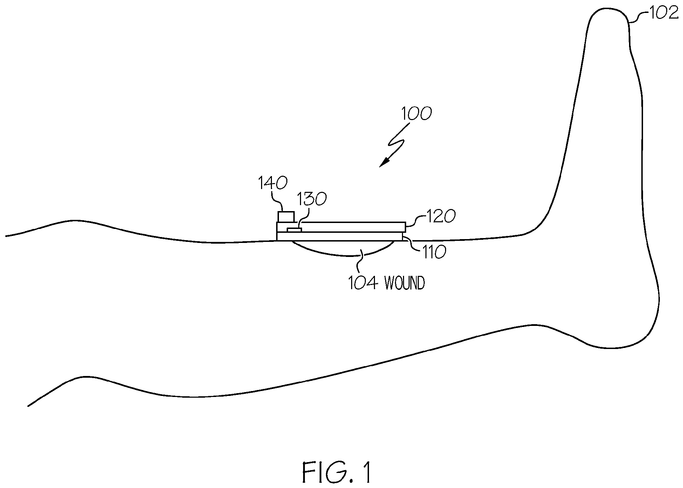

Referring now to FIG. 1, a wound management system 100 according to one or more embodiments is schematically illustrated. The wound management system 100 includes a wound dressing 110, a heating element 120, a temperature sensor 130, and a controller 140. The wound dressing 110 may include, for example, moisture-retentive foam, film, hydrogel, hydrocolloid, or alginate dressings, biologics, skin substitutes, and specifically including dressings that comprise a negative pressure wound therapy (NPWT) system. The wound dressing 110 is configured to cover the wound surface of the wound 104. The wound dressing 110 may seal the wound surface of the wound 104 in order to protect the wound surface from external pathogens. The wound dressing 110 includes a wound facing surface that faces the wound surface of the wound 104 and an outer surface.

In embodiments, the heating element 120 may be coupled to the outer surface of the wound dressing 110. In some embodiments, the heating element 120 may be coupled to the wound facing surface of the wound dressing 110. The heating element 120 is configured to heat the wound 104 of a patient 102 and maintain the wound 104 at a predetermined temperature (e.g., 37 degrees Celsius) or a predetermined temperature range (e.g., 36 degrees Celsius through 38 degrees Celsius). The heating element 120 may be a radiant warmer that transfers heat to the patient 102 via radiant heat transfer, and in some embodiment, may be an infrared heater which emits infrared energy that is absorbed by the patient 102.

The temperature sensor 130 is configured to detect the temperature of the wound 104. The temperature sensor 130 may measure the temperature of the wound surface of the wound 104. The temperature sensor 103 may also measure the temperature of the peri-wound of the wound 104. Although FIG. 1 illustrates that the temperature sensor 130 is placed on the outer surface of the wound dressing 110, the temperature sensor 130 may be placed at the wound facing surface of the wound dressing 110. The temperature sensor 130 may communicate the measured temperature to the controller 140.

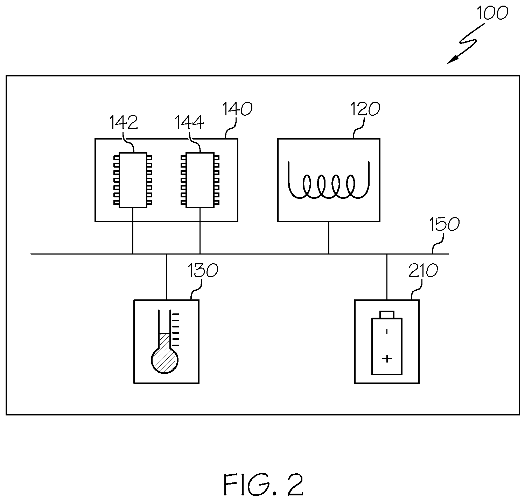

The controller 140 is configured to receive a temperature detected by the temperature sensor 130 and control the operation of the heating element 120 based on the detected temperature. The details of the controller 140 as well as other elements of the wound management system 100 will be described below with reference to FIG. 2.

FIG. 2 schematically depicts the interconnection of various element components of the wound management system 100 including a power element according to one or more embodiments shown and described herein. The wound management system 100 includes the heating element 120, the temperature sensor 130, the controller 140, a communication path 150, and a power element 210. The various components of the wound management system 100 will now be described.

The controller 140 includes one or more processors 142 and one or more memory modules 144 to which various components are communicatively coupled, as will be described in further detail below. In some embodiments, the one or more processors 142 and the one or more memory modules 144 and/or the other components are included within a single device. In other embodiments, the one or more processors 142, the one or more memory modules 144 and/or the other components may be distributed among multiple devices that are communicatively coupled. For example, the one or more processors 142, and the one or more memory modules 144 are included in a remote device that wirelessly communicates with other elements, e.g., the heating element 120 and the temperature sensor 130.

The controller 140 includes the one or more memory modules 144 that store a set of machine readable instructions. The one or more processors 142 execute the machine readable instructions stored in the one or more memory modules 144. The one or more memory modules 144 may comprise RAM, ROM, flash memories, hard drives, or any device capable of storing machine readable instructions such that the machine readable instructions can be accessed by the one or more processors 142. The machine readable instructions comprise logic or algorithm(s) written in any programming language of any generation (e.g., 1GL, 2GL, 3GL, 4GL, or 5GL) such as, for example, machine language that may be directly executed by the one or more processors 142, or assembly language, object-oriented programming (OOP), scripting languages, microcode, etc., that may be compiled or assembled into machine readable instructions and stored in the one or more memory modules 144. Alternatively, the machine readable instructions may be written in a hardware description language (HDL), such as logic implemented via either a field-programmable gate array (FPGA) configuration or an application-specific integrated circuit (ASIC), or their equivalents. Accordingly, the methods described herein may be implemented in any conventional computer programming language, as pre-programmed hardware elements, or as a combination of hardware and software components. The one or more memory modules 144 may be implemented as one memory module or a plurality of memory modules.

The one or more memory modules 144 include instructions for executing the functions of the wound management system 100. The instructions may include instructions for receiving a temperature measured by the temperature sensor 130, instructions for determining whether the temperature is lower than a predetermined temperature (e.g., 36 °C), and instructions for
activating the heating element 120 in response to determination that the temperature is lower than the predetermined temperature. In some embodiments, the wound management system 100 activates the power element 210 to operate the heating element 120 when it is determined that the temperature is lower than the predetermined temperature. The instructions may further include instructions for determining whether the temperature detected by the temperature sensor 130 is higher than a second predetermined temperature (e.g., 38 °C), and instructions for deactivating the heating element 120 in response to determination that the temperature is higher than the second predetermined temperature.

The one or more processors 142 may be any device capable of executing machine readable instructions. For example, the one or more processors 142 may be an integrated circuit, a microchip, a computer, or any other computing device. The one or more memory modules 144 and the one or more processors 142 are coupled to a communication path 150 that provides signal interconnectivity between various components and/or modules of the wound management system 100. Accordingly, the communication path 150 may communicatively couple any number of processors with one another, and allow the modules coupled to the communication path 150 to operate in a distributed computing environment. Specifically, each of the modules may operate as a node that may send and/or receive data. As used herein, the term "communicatively coupled" means that coupled components are capable of exchanging data signals with one another such as, for example, electrical signals via conductive medium, electromagnetic signals via air, optical signals via optical waveguides, and the like.

Accordingly, the communication path 150 may be formed from any medium that is capable of transmitting a signal such as, for example, conductive wires, conductive traces, optical waveguides, or the like. Moreover, the communication path 150 may be formed from a combination of mediums capable of transmitting signals. In some embodiments, the communication path 150 comprises a combination of conductive traces, conductive wires, connectors, and buses that cooperate to permit the transmission of electrical data signals to components such as processors, memories, sensors, input devices, output devices, and communication devices. Additionally, it is noted that the term "signal" means a waveform (e.g., electrical, optical, magnetic, mechanical or electromagnetic), such as DC, AC, sinusoidal-wave, triangular-wave, square-wave, vibration, and the like, capable of traveling through a medium.

The heating element 120 is coupled to the communication path 150 and communicatively coupled to the controller 140 and the power element 210. The heating element 120 is configured to heat the wound 104 of the patient 102 (See FIG. 1) and maintain the wound at a predetermined temperature or a predetermined temperature range (e.g., 36 °C through 38 °C). The heating element 120 may be a radiant warmer that transfers heat to the patient 102 via radiant heat transfer, particularly, an infrared heater which emits infrared energy that is absorbed by the patient 102.

The temperature sensor 130 is coupled to the communication path 150 and communicatively coupled to the controller 140. The temperature sensor 130 is configured to measure the temperature of the wound 104. The temperature sensor 130 may measure the temperature of the wound surface of the wound 104. The temperature sensor 103 may also measure the temperature of the peri-wound of the wound 104. In some embodiments, the temperature sensor 130 may wirelessly transmit the measured temperature to the controller 140.

The power element 210 is configured to provide a power to the heating element 120, and in some embodiments, the other element components of the wound management system 100. The power element may include a disposable battery, rechargeable or replicable battery, a wired power source, a power source that wirelessly transmits power or transmits power through exothermic reaction, or a photoelectric power element. When the controller 140 determines that the wound surface of the wound 104 should be heated, it causes the power element 210 to provide power to the heating element 120 and activate the heating element 120. Specifically, when the temperature measured by the temperature sensor 130 is lower than a predetermined temperature, the controller 140 causes the power element 210 to provide power to the heating element 120 and activate the heating element 120. In some embodiments, the wound management system 100 may not include the temperature sensor 130, i.e., an open loop system. With respect to the open loop system, the controller 140 causes the power element 210 to provide power to the heating element 120 constantly, or intermittently to achieve a desired level of heating.

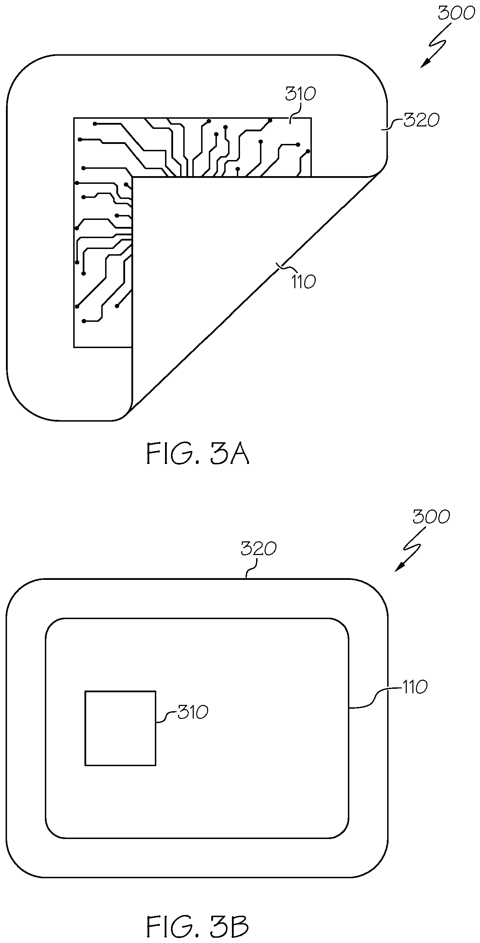

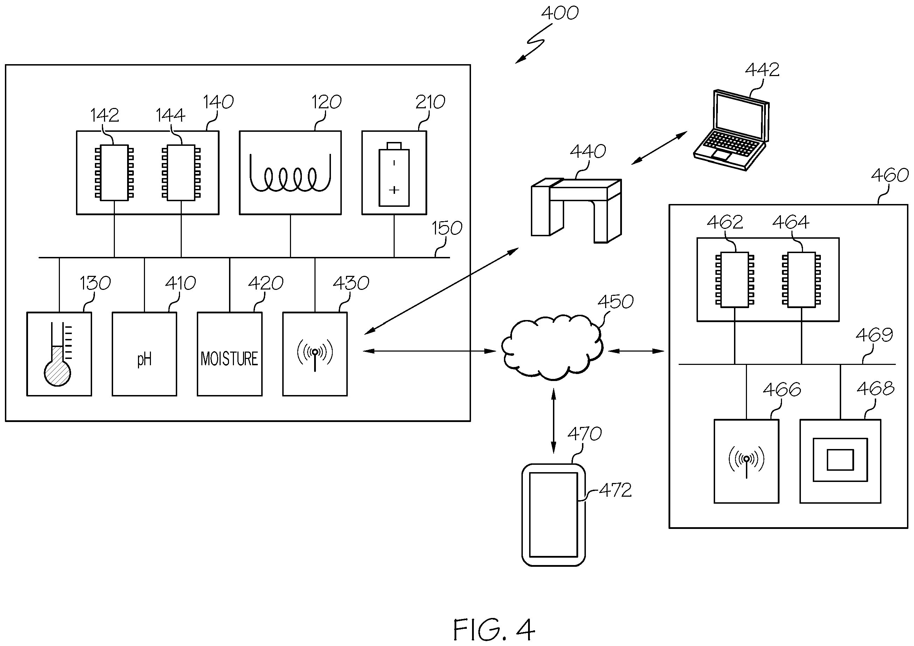

FIGS. 3A and 3B depict an exemplary wound management system 300 including one or more sensing devices 310 according to one or more embodiments shown and described herein. The wound management system 300 includes one or more sensing devices 310, a bandage 320, and a wound dressing 110. FIG. 3A depicts a bandage 320 that includes a wound dressing 110 at the bottom of the bandage 320, and one or more sensing devices 310 on top of the bandage 320. In some embodiments, the one or more sensing devices 310 may be placed between the bandage 320 and the wound dressing 110. In some embodiments, the bandage 320 includes one or more adhesive areas that may be attached to the perimeter surrounding a wound surface. FIG. 3B depicts a bandage 320 that includes a wound dressing 110 as well as the one or more sensing devices 310 at the bottom of the bandage 320. The one or more sensing devices 310 may include the temperature sensor 130, a pH meter, a moisture sensor, etc. Details of the one or more sensing devices 310 and other elements will be described below with reference to FIG. 4.

FIG. 4 schematically depicts an exemplary wound management system 400 including one or more sensing devices according to one or more embodiments shown and described herein. The wound management system 400 includes the heating element 120, the temperature sensor 130, the controller 140, the power element 210, a pH meter 410, a moisture sensor 420, and a network interface hardware 430. The various components of the wound management system 400 will now be described.

The controller 140 includes one or more processors 142 and one or more memory modules 144 to which various components are communicatively coupled, as will be described in further detail below. In some embodiments, the one or more processors 142 and the one or more memory modules 144 and/or the other components are included within a single device. In other embodiments, the one or more processors 142, the one or more memory modules 144 and/or the other components may be distributed among multiple devices that are communicatively coupled. For example, in some embodiments, the one or more processors 142 and the one or more memory modules 144 are included in a remote device that wirelessly communicates with other elements, e.g., the heating element 120 and the temperature sensor 130.

The controller 140 includes the one or more memory modules 144 that store a set of machine readable instructions. The one or more processors 142 execute the machine readable instructions stored in the one or more memory modules 144. The one or more memory modules 144 may comprise RAM, ROM, flash memories, hard drives, or any device capable of storing machine readable instructions such that the machine readable instructions can be accessed by the one or more processors 142. The machine readable instructions comprise logic or algorithm(s) written in any programming language of any generation (e.g., 1GL, 2GL, 3GL, 4GL, or 5GL) such as, for example, machine language that may be directly executed by the one or more processors 142, or assembly language, object-oriented programming (OOP), scripting languages, microcode, etc., that may be compiled or assembled into machine readable instructions and stored in the one or more memory modules 144. Alternatively, the machine readable instructions may be written in a hardware description language (HDL), such as logic implemented via either a field-programmable gate array (FPGA) configuration or an application-specific integrated circuit (ASIC), or their equivalents. Accordingly, the methods described herein may be implemented in any conventional computer programming language, as pre-programmed hardware elements, or as a combination of hardware and software components. The one or more memory modules 144 may be implemented as one memory module or a plurality of memory modules.

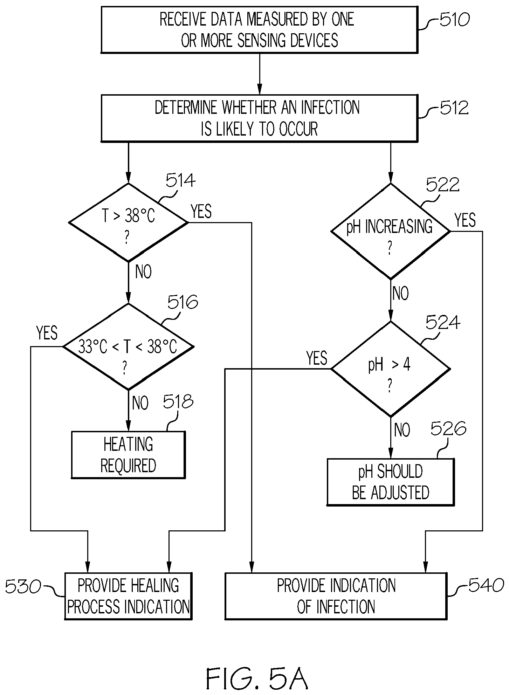

The one or more memory modules 144 include instructions for executing the functions of the wound management system 400. The instructions may include instructions for receiving data measured by the one or more sensing devices, instructions for determining whether an infection is likely to occur based on the data measured by the one or more sensing devices, and instructions for outputting an alert, through the network interface hardware 430, in response to determination that the infection is likely to occur. The one or more sensing devices may include at least one of the temperature sensor 130, the pH meter 410, and the moisture sensor 420. Details of determining whether an infection is likely to occur will be described below with reference to FIG. 5A. The one or more memory modules 144 may store identification information of the wound management system 400.

The one or more processors 142 may be any device capable of executing machine readable instructions. For example, the one or more processors 142 may be an integrated circuit, a microchip, a computer, or any other computing device. The one or more memory modules 144 and the one or more processors 142 are coupled to a communication path 150 that provides signal interconnectivity between various components and/or modules of the wound management system 400. Accordingly, the communication path 150 may communicatively couple any number of processors with one another, and allow the modules coupled to the communication path 150 to operate in a distributed computing environment. Specifically, each of the modules may operate as a node that may send and/or receive data. As used herein, the term "communicatively coupled" means that coupled components are capable of exchanging data signals with one another such as, for example, electrical signals via conductive medium, electromagnetic signals via air, optical signals via optical waveguides, and the like.

Accordingly, the communication path 150 may be formed from any medium that is capable of transmitting a signal such as, for example, conductive wires, conductive traces, optical waveguides, or the like. Moreover, the communication path 150 may be formed from a combination of mediums capable of transmitting signals. In some embodiments, the communication path 150 comprises a combination of conductive traces, conductive wires, connectors, and buses that cooperate to permit the transmission of electrical data signals to components such as processors, memories, sensors, input devices, output devices, and communication devices. Additionally, it is noted that the term "signal" means a waveform (e.g., electrical, optical, magnetic, mechanical or electromagnetic), such as DC, AC, sinusoidal-wave, triangular-wave, square-wave, vibration, and the like, capable of traveling through a medium.

The heating element 120 is coupled to the communication path 150 and communicatively coupled to the controller 140 and the power element 210. The heating element 120 is configured to heat a wound 104 of a patient 102 (See FIG. 1) and maintain the wound at a predetermined temperature or a predetermined temperature range (e.g., 36 °C through 38 °C). The heating element 120 may be a radiant warmer that transfers heat to the patient 102 via radiant heat transfer, particularly, an infrared heater which emits infrared energy that is absorbed by the patient 102. In some embodiments, the wound management system 400 may not include the heating element 120.

The temperature sensor 130 is coupled to the communication path 150 and communicatively coupled to the controller 140. The temperature sensor 130 is configured to measure the temperature of the wound 104 in FIG. 1. The temperature sensor 103 may also measure the temperature of the peri-wound of the wound 104. The temperature sensor 130 may transmit the measured temperature to the controller 140 through the communication path 150. In some embodiments, the temperature sensor 130 may wirelessly transmit measured temperature to the controller 140. The temperature sensor 130 may be placed on the top of the bandage 320 as shown in FIG. 3A, or at the bottom of the wound dressing 110 as shown in FIG. 3B. The measured temperature may be stored in the one or more memory modules 144. In some embodiments, the measured temperature may be stored along with the time of measuring in the one or more memory modules.

The pH meter 410 is coupled to the communication path 150 and communicatively coupled to the controller 140. The pH meter 410 is configured to measure the pH of the wound 104. The pH meter 410 may transmit the measured pH to the controller 140 through the communication path 150. In some embodiments, the pH meter 410 may wirelessly transmit measured pH to the controller 140. The pH meter 410 may be placed on the top of the bandage 320 as shown in FIG. 3A, or at the bottom of the wound dressing 110 as shown in FIG. 3B. The measured pH may be stored in the one or more memory modules 144. In some embodiments, the measured pH may be stored along with the time of measuring in the one or more memory modules 144.

The moisture sensor 420 is coupled to the communication path 150 and communicatively coupled to the controller 140. The moisture sensor 420 is configured to measure the moisture level of the wound. The moisture sensor 420 may transmit the measured moisture level to the controller 140 through the communication path 150. In some embodiments, the moisture sensor 420 may wirelessly transmit measured level of moisture to the controller 140. The moisture sensor 420 may be placed on the top of the bandage 320 as shown in FIG. 3A, or at the bottom of the wound dressing 110 as shown in FIG. 3B. The measured moisture level may be stored in the one or more memory modules 144.

In some embodiments, the wound management system 400 may include a biomarker sensor for detecting biomarkers of infection of healing of wounds. The biomarker sensor may be an imaging sensor, or a combination of the imaging sensor, the temperature sensor 130, the pH meter 410, and/or the moisture sensor 420. The biomarker sensor may detect biomarkers of infection, for example, pathogens and active signs of infection. The biomarker sensor may detect biomarkers that a wound is healing or not healing. In some embodiments, the controller 140 receives data from the biomarker sensor and determines whether the data indicates a sign of infection or a sign of healing.

The power element 210 is configured to provide a power to the heating element 120 and, in some embodiments, other components of the wound management system 400. The power element may include a disposable battery, a rechargeable or replicable battery, a wired power source, and a power source that wirelessly transmits power or transmits power through exothermic reaction. When the controller 140 determines that the wound surface of the wound 104 should be heated, it instructs the power element 210 to provide power to the heating element 120 and activate the heating element 120. Specifically, when the temperature detected by the temperature sensor 130 is lower than a predetermined temperature, the controller 140 instructs the power element 210 to provide power to the heating element 120 and activate the heating element 120. In some embodiments, the wound management system 400 may not include the temperature sensor 130, i.e., an open loop system. For the open loop system, the controller 140 instructs the power element 210 to provide power to the heating element 120 constantly, or intermittently to achieve a desired level of heating. In some embodiments, the wound management system 400 may not include the power element 210.

The network interface hardware 430 is coupled to the communication path 150 and communicatively coupled to the controller 140. The network interface hardware 430 can be communicatively coupled to the communication path 150 and can be any device capable of transmitting and/or receiving data to and from an external device such as a RFID reader 440, a remote server 460, or a smart phone 470. Accordingly, the network interface hardware 430 can include a communication transceiver for sending and/or receiving any wired or wireless communication. For example, the network interface hardware 430 may include an antenna, a modem, LAN port, Wi-Fi card, WiMax card, an RFID transmitter, mobile communications hardware, near-field communication hardware, satellite communication hardware and/or any wired or wireless hardware for communicating with other networks and/or devices. In one embodiment, the network interface hardware 430 includes hardware configured to operate in accordance with the Bluetooth wireless communication protocol.

The server 460 may be communicatively coupled to the wound management system 400 by a network 450. In one embodiment, the network 450 may include one or more computer networks (e.g., a personal area network, a local area network, or a wide area network), cellular networks, satellite networks and/or a global positioning system and combinations thereof. Accordingly, the server 460 and the wound management system 400 can be communicatively coupled to the network 450 via a wide area network, via a local area network, via a personal area network, via a cellular network, via a satellite network, etc. Suitable local area networks may include wired Ethernet and/or wireless technologies such as, for example, wireless fidelity (Wi-Fi). Suitable personal area networks may include wireless technologies such as, for example, IrDA, Bluetooth, Wireless USB, Z-Wave, ZigBee, and/or other near field communication protocols. Suitable cellular networks include, but are not limited to, technologies such as LTE, WiMAX, UMTS, CDMA, and GSM.

The server 460 may include one or more processors 462, one or more memory modules 464, a network interface hardware 466, a display 468, and a communication path 469. The one or more processors 462 may be processors similar to the one or more processors 142 described above. The one or more memory modules 464 may be memories similar to the one or more memory modules 144 described above. The network interface hardware 466 may be interface hardware similar to the network interface hardware 430 described above. The communication path 469 may be a communication path similar to the communication path 150 described above. The display 468 may include any medium capable of transmitting an optical output such as, for example, a cathode ray tube, a light emitting diode (LED) display, an organic light emitting diode (OLED) display, a liquid crystal display, a plasma display, or the like.

The one or more processors 462 can execute logic to communicate with the wound management system 400. The server 460 may be configured with wired and/or wireless communication functionality for communicating with the wound management system 400. In some embodiments, the server 460 may perform one or more elements of the functionality described herein, such as in embodiments in which the functionality described herein is distributed between the wound management system 400 and the server 460. In some embodiments, the server 460 may provide a user interface through which one or more settings or configurations of the wound management system 400 may be altered. For example, the server 460 may provide a user interface for setting a desired temperature range of the wound management system 400. The user may enter, e.g., the range of 36 °C to 38 °C through the user interface.

In embodiments, the server 460 may communicate with a plurality of wound management systems 400. The one or more memory modules 464 may include a database for a plurality of wound management systems. An exemplary database is shown in Table 1 below.

**Table 1**

| ID | Time | Temperature (°C) | pH | Moisture | Location |
|---|---|---|---|---|---|
| 1 | 8/10/2017 2:01:00 AM | 36.5 | 6.3 | 80% | Room 101 |
| 2 | 8/10/2017 2:01:00 AM | 38.1 | 7.1 | 92% | Room 102 |
| 3 | 8/10/2017 2:01:00 AM | 37.2 | 5.2 | 54% | Room 103 |
| 1 | 8/10/2017 2:11:00 AM | 36.6 | 7.3 | 81% | Room 101 |
| 2 | 8/10/2017 2:11:00 AM | 38.3 | 7.1 | 92% | Room 102 |
| 3 | 8/10/2017 2:11:00 AM | 37.2 | 5.2 | 55% | Room 103 |

The database may include an ID for each of the plurality of the wound management systems. The database may also include the time that the server 460 received data from each of the plurality of the wound management systems. In some embodiments, each of the wound management systems records time when the temperature, pH, and/or the moisture level are measured, and transmits the time along with the measured data to the server 460. The database may also include the location (e.g., a patient Room number) of each of the plurality of the wound management systems. The location of each of the plurality of the wound management systems may be received from each of the plurality of the wound management systems. The location of each of the plurality of the wound management systems may be stored in each of the plurality of the wound management systems using, for example, GPS of the wound management system. In some embodiments, a health care provide may input the location of each of the plurality of wound management systems into the database after delivering the wound management system to a certain location (e.g., Room 101).

The display 468 may display information about the status of the wound management system 400. For example, the display 468 may display identification information about the wound management system 400, the temperature measured by the temperature sensor 130, the pH measured by the pH meter 410, and/or the moisture level measured by the moisture sensor 420. The display 468 may also display the risk of infection based on the data including the temperature, pH, and/or the moisture level.

The RFID reader 440 may read information stored in the wound management system 400 (e.g., by communicating with the network interface hardware 430). Specifically, the RFID reader 440 may read identification information about the wound management system 400, the temperature measured by the temperature sensor 130, the pH measured by the pH meter 410, and/or the moisture level measured by the moisture sensor 420. The RFID reader 440 is communicatively coupled to a computing device 442, and transmits data including the temperature, pH, and/or moisture level to the computing device 442. The computing device 442 may include one or more processors, one or more memory modules, a network interface hardware, and a display similar to the server 460. A health care provider can check the status of the wound management system 400 by accessing the computing device 442 and monitoring data including temperature, pH, and/or moisture level received from the wound management system 400. The display of the computing device 442 may display the risk of infection based on the data including the temperature, pH, and/or the moisture level. For example, the display of the computing device 442 may indicate "Temperature for ID No. 2 is over 38 °C. Infection is likely to occur in ID No. 2."

The smart phone 470 may be communicatively coupled to the wound management system 400 by the network 450. The smart phone 470 may include one or more processors, one or more memory modules, a network interface hardware, and a display 472 similar to the server 460. The display 472 of the smart phone 470 may display information about the status of the wound management system 400. For example, the display 472 may display identification information about the wound management system 400, the temperature measured by the temperature sensor 130, the pH measured by the pH meter 410, and/or the moisture level measured by the moisture sensor 420. The display 472 may also display the risk of infection based on the data including the temperature, pH, and/or the moisture level. For example, the display 472 may indicate "pH in ID No. 1 is increasing. Infection is likely to occur in ID No. 1." A health care provider can check the status of the wound management system 400 by monitoring data including temperature, pH, and/or moisture level received from the wound management system 400 that is displayed on the display 472.

FIG. 5A depicts a flowchart for determining whether an infection is likely to occur, according to one or more embodiments shown and described herein. In step 510, the controller 140 of the wound management system 400 receives data measured by one or more sensing devices including the temperature sensor 130, the pH meter 410, and/or the moisture sensor 420.

In step 512, the controller 140 of the wound management system 400 determines whether an infection is likely to occur in the wound 104 based on the data measured by one or more sensing devices including the temperature sensor 130, the pH meter 410, and/or the moisture sensor 420. For example, in step 514, the controller 140 may determine whether the temperature measured by the temperature sensor 130 is higher than a predetermined temperature, e.g., 38 degrees Celsius. If it is determined that the temperature measured by the temperature sensor 130 is higher than the predetermined temperature, the controller 140 provides an indication of infection in step 540. The controller 140 may send an alert message to the server 460, the computing device 442, and/or the smart phone 470 through the network interface hardware 430 to provide the indication of the infection.

If it is determined that the temperature measured by the temperature sensor 130 is not higher than 38 degrees Celsius in step 514, then the controller 140 determines whether the temperature measured by the temperature sensor 130 is within a predetermined range, for example, between 33 degrees Celsius and 38 degrees Celsius, in step 516. If it is determined that the temperature measured by the temperature sensor 130 is within the predetermined range in step 516, the controller 140 provides an indication the wound 104 is in the process of healing, in step 530. The controller 140 may send a message indicating that the wound 104 is in the process of healing to the server 460, the computing device 442, and/or the smart phone 470 through the network interface hardware 430. In some embodiments, if it is determined that the temperature measured by the temperature sensor 130 is within a second predetermined range, e.g., between 34 degrees Celsius and 36 degrees Celsius, the controller 140 provides an indication that the wound 104 is in health inflammation, which is the sign of tissue healing. The controller 140 may send a message indicating that the wound is in health inflammation to the server 460, the computing device 442, and/or the smart phone 470 through the network interface hardware 430.

If it is determined that the temperature measured by the temperature sensor 130 is not within the predetermined range, for example, the temperature is lower than 33 degrees Celsius at step 516, the controller 140 determines that heating is required in step 518. The controller 140 may cause the power element 210 to provide power to the heating element 120 in step 518 and activate the heating element 120, as described above with reference to FIG. 2.

In step 522, the controller 140 determines whether the pH measured by the pH meter 410 is increasing. If it is determined that the pH measured by the pH meter 410 is increasing, e.g., from pH 7 to pH 8 during the last one hour, the controller 140 provides an indication of infection in step 540. The controller 140 may send an alert message to the server 460, the computing device 442, and/or the smart phone 470 through the network interface hardware 430 to provide the indication of infection.

If it is determined that the pH measured by the pH meter 410 is not increasing at step 522, the controller 140 determines whether the pH is higher than a predetermined value (e.g., pH 4), in step 524. If it is determined that the pH is higher than 4 in step 524, the controller 140 notifies the wound 104 is in the process of healing, in step 530. The controller 140 may send a message indicating that the wound 104 is in the process of healing to the server 460, the computing device 442, and/or the smart phone 470 through the network interface hardware 430. If it is determined that the pH is not higher than 4 at step 524, the controller 140 provides an indication that the pH of the wound management system 400 should be adjusted, in step 526.

Although the process described above is implemented by the controller 140 of the wound management system 400, the process may be implemented by the one or more processors 462 of the server 460, or one or more processors of the computing device 442 or the smart phone 470. In embodiments, the one or more processors of the server 460 receive the data measured by the one or more sensing devices, and the one or more processors 462 of the server 460 determine whether an infection is likely to occur. For example, in Table 1 above, the temperature from the system ID 2 is 38.1 degrees Celsius at 2:01:00 AM, August 10, 2017. Because the temperature is higher than 38 degrees Celsius, the one or more processors 462 may output an alert message, for example, display an alert message on the display 468 indicating "The temperature for the system ID 2 is over 38 °C. An infection is likely to occur for the system ID 2." As another example, in Table 1 above, the pH from the system ID 1 increased from pH 6.3 to pH 7.3 between 2:01:00 AM, August 10, 2017 and 2:11:00 AM, August 10, 2017. Because the pH is increasing, the one or more processors 462 may output an alert message, for example, display an alert message on the display 468 indicating "pH for system ID 1 is increasing. An infection is likely to occur for the system ID 1."

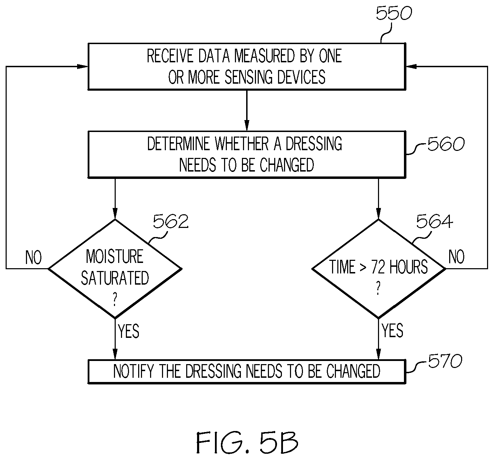

FIG. 5B depicts a flowchart for determining whether a dressing needs to be changed, according to one or more embodiments shown and described herein. In step 550, the controller 140 of the wound management system 400 receives data measured by one or more sensing devices including the temperature sensor 130, the pH meter 410, and/or the moisture sensor 420.

In step 560, the controller 140 of the wound management system 400 determines whether the wound dressing 110 needs to be changed based on the data measured by one or more sensing devices including the temperature sensor 130, the pH meter 410, and/or the moisture sensor 420. For example, in step 562, the controller 140 may determine whether the moisture level measured by the moisture sensor 420 is saturated. If it is determined that the moisture level measured by the moisture sensor 420 is saturated at step 562, the controller 140 provides a notification that the wound dressing 110 needs to be changed, in step 570. The controller 140 may send an alert message to the server 460, the computing device 442, and/or the smart phone 470 through the network interface hardware 430, indicating that the wound dressing 110 needs to be changed. If it is determined that the moisture level measured by the moisture sensor 420 is not saturated at step 562, the process returns to the step 550 and the controller 140 continues to receive data measured by the moisture sensor 420.

In step 564, the controller 140 may determine whether the wound dressing 110 has been placed over the wound 104 more than a predetermined time, for example, 72 hours. The one or more memory modules 144 may store the time when the wound dressing 110 is placed on the wound 104. For example, a health care provide may write time to the wound management system 400 (e.g., writing time to a RFID tag of the wound management system 400 or inputting time to the one or more memory modules 144) when she places the wound dressing 110 on the wound. If it is determined that the wound dressing 110 has been placed over the wound 104 more than the predetermined time at step 562, the controller 140 provides a notification that the wound dressing 110 needs to be changed, in step 570. The controller 140 may send an alert message to the server 460, the computing device 442, and/or the smart phone 470 through the network interface hardware 430, indicating that the wound dressing 110 needs to be changed. If it is determined that the wound dressing 110 has not been placed over the wound 104 more than predetermined hours, the process returns to step 550.

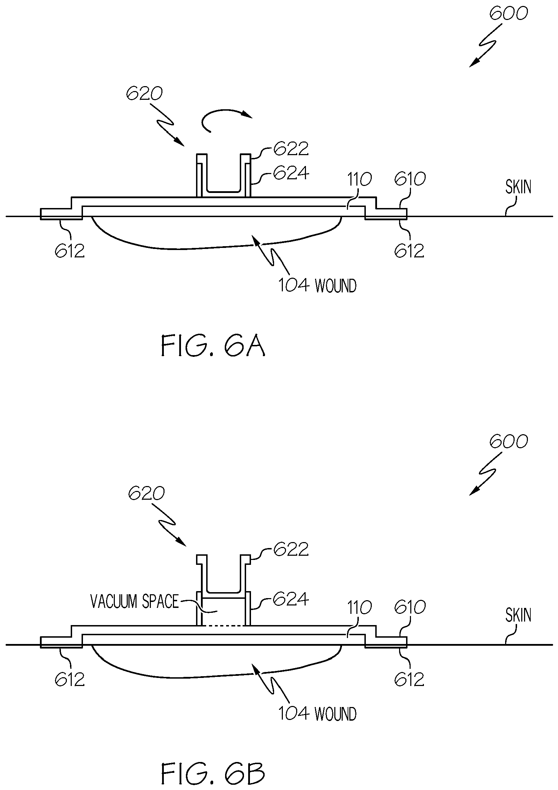

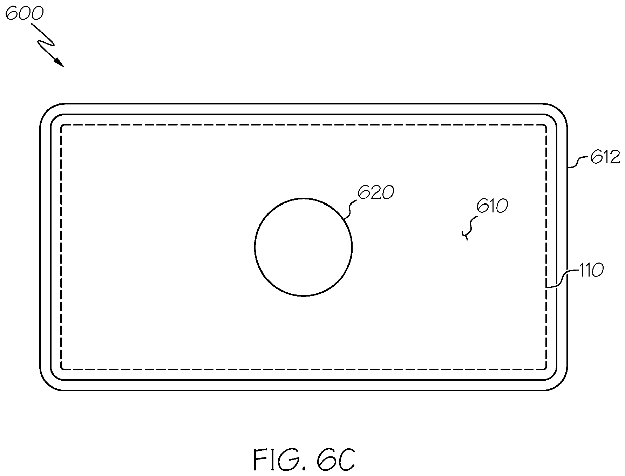

FIGS. 6A and 6B depict a wound protection system having a knob configured to create a vacuum seal, according to one or more embodiments shown and described herein. The wound protection system 600 includes the wound dressing 110, an outer layer 610, and a mechanical actuator 620 (e.g., a rotating knob). The wound dressing 110 may be attached at the bottom of the outer layer 610 and configured to cover the wound 104. The outer layer 610 includes an adhesive border 612 which can be attached to the skin of a patient. FIG. 6C depicts a top view of the wound protection system 600, and the adhesive border 612 is a closed loop such that it sealed the wound surface of the wound 104.

The mechanical actuator 620 is attached on top of the outer layer 610. In embodiments, the mechanical actuator 620 includes an upper portion 622 and a lower portion 624. In embodiments, the upper portion 622 and the lower portion 624 are nested (for example, as paired wedges, mated tracks, or screw-driven) such that when the upper portion 622 is rotated, the upper portion 622 and the lower portion 624 are moved apart, as shown in FIG. 6B. The opening between the upper portion 622 and the lower portion 624 creates a volume that creates a vacuum between the outer layer 610 and the wound surface. The vacuum created by the mechanical actuator 620 confirms that a barrier for the wound has been created.

Although FIGS. 6A and 6B depict the specific structure of the upper portion 622 and the lower portion 624 of the mechanical actuator 620, any other mechanisms that create a vacuum seal may be used. In some embodiments, the mechanical actuator 620 may include a container (e.g., a cup body), a rotating knob attached to the top of the container, and a plunger constituting the bottom of the container. The plunger may be in contact with the wound dressing 110 in its original position similar to the bottom of the upper portion 622 in FIG. 6A. When the rotating knob is rotated, the plunger is elevated to create a vacuum seal between the outer layer 610 and the wound surface. In some embodiments, the mechanical actuator 620 includes an air valve and an external pump which create a vacuum seal between the outer layer 610 and the wound surface.

In some embodiments, when the vacuum created by the mechanical actuator 620 is broken, a visible change in the mechanical actuator 620 occurs, such that a patient or a health care provider knows that the barrier for the wound is no longer intact. For example, the upper portion 622 may have a concave surface when the vacuum seal is intact. If the vacuum is broken, the concave surface changes to a convex surface, which is visible to the patient or the health care provider.

Embodiments described herein include a wound monitoring system includes a wound dressing configured to cover a wound surface of the wound, one or more sensing devices attached to the wound dressing, a network interface hardware, and a controller. The controller includes one or more processors, and one or more memory modules storing computer readable and executable instructions. The controller receives data measured by the one or more sensing devices, determines whether an infection is likely to occur based on the data measured by the one or more sensing devices, and outputs an alert, through the network interface hardware, in response to determining that the infection is likely to occur.

By collecting and transmitting data measured by one or more sensing devices, the wound monitoring system prevents unnecessary disturbance of dressing. In addition, the wound monitoring system allows maintenance of an optimized moist wound environment by using a moisture sensor. Furthermore, the wound monitoring system eliminates unnecessary dressing changes, which in turn reduces pain experienced by the patient, and prevents heat loss resulting from dressing change. The wound monitoring system also allows maintenance of a waterproof wound environment, which allows a patient to bath.

It will be apparent to those skilled in the art that various modifications and variations can be made to the embodiments described herein.

## Claims

1. A system (100) for monitoring a wound (104) comprising:
a wound dressing (110) configured to cover a wound surface of the wound (104);
one or more sensing devices attached to the wound dressing (110), the one or more sensing devices comprising a pH meter (410);
a network interface hardware (430); and
a controller (140) comprising:
one or more processors (142); and
one or more memory modules (144) storing computer readable and executable instructions which, when executed by the one or more processors (142), cause the controller (140) to:
receive data measured by the one or more sensing devices along with times of measuring;
determine whether a level of pH measured by the pH meter (410) is increasing over time based on the data and times of measuring;
determine that an infection is likely to occur when the level of the pH measured by the pH meter (410) increases over time; and
output an alert, through the network interface hardware (430), in response to determining that the infection is likely to occur.

2. The system (100) of claim 1, wherein the one or more sensing devices include a temperature sensor (130), and
the computer readable and executable instructions cause the controller (140) to:
determine whether a temperature measured by the temperature sensor (130) is higher than a first predetermined temperature; and
determine that the infection is likely to occur in response to determination that the temperature is higher than the first predetermined temperature.

3. The system (100) of claim 2, wherein the first predetermined temperature is 38 degrees Celsius.

4. The system (100) of any preceding claim, wherein the one or more sensing devices include a temperature sensor (130), and
the computer readable and executable instructions cause the controller (140) to:
determine whether a temperature measured by the temperature sensor (130) is within a predetermined range; and
output a message indicating that the wound (104) is in the process of healing in response to determination that the temperature is within the predetermined range.

5. The system (100) of claim 4, wherein the predetermined range is a range between 33 degrees Celsius and 37 degrees Celsius.

6. The system (100) of any preceding claim, wherein the one or more sensing devices include a moisture sensor (420), and
the computer readable and executable instructions cause the controller (140) to:
determine whether a saturation occurs in the wound dressing (110) based on a moisture level measured by the moisture sensor (420); and
output an alert, through the network interface hardware (430), in response to determination that the saturation occurs in the wound dressing (110).

7. The system (100) of any preceding claim, wherein the one or more memory modules (144) store a time when the wound dressing (110) is first used, and
the computer readable and executable instructions cause the system (100) to:
determine whether the wound dressing (110) has been used for a time longer than a predetermined time based on the time when the wound dressing (110) is first used; and
output an alert in response to determination that the wound dressing (110) has been used longer than a predetermined time.

8. The system (100) of claim 7, wherein the predetermined time is 72 hours.

9. The system (100) of any preceding claim, wherein the one or more sensing devices include a biomarker sensor configured to detect a sign of infection or a sign of healing for the wound (104).

10. The system (100) of any preceding claim, wherein the wound dressing (110) includes a wound facing surface configured to face the wound surface and an outer surface, and the one or more sensing devices is attached on the wound facing surface.

11. The system (100) of any preceding claim, further comprising a heating element attached to the wound dressing (110),
wherein:
the one or more sensing devices include a temperature sensor (130) configured to measure a temperature, and
the computer readable and executable instructions cause the controller (140) to activate the heating element (120) in response to the temperature being lower than a desired predetermined temperature.

12. The system (100) of claim 11, further comprising a power element (210) configured to activate the heating element (120) in response to the temperature being lower than the desired predetermined temperature.

13. The system (100) of claim 12, wherein the power element (210) includes a photoelectric power element.

14. The system (100) of claim 13, wherein the second predetermined temperature is 36 degrees Celsius.

## Patentansprüche

1. Ein System (100) zur Überwachung einer Wunde (104), umfassend:
einen Wundverband (110), der so konfiguriert ist, dass eine Wundoberfläche der Wunde (104) bedeckt wird;
eine oder mehrere an dem Wundverband (110) angebrachte Sensorvorrichtung(en), wobei die eine oder mehreren Sensorvorrichtung(en) ein pH-Messgerät (410) umfasst/umfassen;
eine Netzwerkschnittstellen-Hardware (430); und
eine Steuereinheit (140) umfassend:
einen oder mehrere Prozessor(en) (142); und
ein oder mehrere Speichermodul(e) (144), worin vom Computer lesbare und ausführbare Befehle gespeichert sind, die bei Ausführung durch den einen oder mehrere Prozessor(en) (142) die Steuereinheit (140) dazu veranlassen:
von der einen oder den mehreren Sensorvorrichtung(en) gemessene Daten zusammen mit den Messzeiten zu empfangen;
basierend auf den Messdaten und -zeiten festzustellen, ob ein vom pH-Messgerät (410) gemessener pH-Wert im Laufe der Zeit zunimmt;
festzustellen, dass eine Infektion wahrscheinlich auftritt, wenn der vom pH-Messgerät (410) gemessene pH-Wert im Laufe der Zeit ansteigt; und
als Reaktion auf die Feststellung, dass die Infektion wahrscheinlich auftritt, eine Warnmeldung über die Netzwerkschnittstellen-Hardware (430) auszugeben.

2. Das System (100) nach Anspruch 1, wobei die eine oder die mehreren Sensorvorrichtung(en) einen Temperatursensor (130) beinhaltet/beinhalten, und
die vom Computer lesbaren und ausführbaren Befehle die Steuereinheit (140) dazu veranlassen:
festzustellen, ob eine vom Temperatursensor (130) gemessene Temperatur höher ist als eine erste vorgegebene Temperatur; und
als Reaktion auf die Feststellung, dass die Temperatur höher als die erste vorgegebene Temperatur ist, festzustellen, dass die Infektion wahrscheinlich auftritt.

3. Das System (100) nach Anspruch 2, wobei die erste vorgegebene Temperatur 38 Grad Celsius beträgt.

4. Das System (100) nach einem der vorstehenden Ansprüche, wobei die eine oder die mehreren Sensorvorrichtung(en) einen Temperatursensor (130) beinhaltet/beinhalten, und
die vom Computer lesbaren und ausführbaren Befehle die Steuereinheit (140) dazu veranlassen:
festzustellen, ob eine vom Temperatursensor (130) gemessene Temperatur innerhalb eines vorgegebenen Bereichs liegt; und
als Reaktion auf die Feststellung, dass die Temperatur innerhalb des vorgegebenen Bereichs liegt, eine Meldung auszugeben, die darauf hinweist, dass sich die Wunde (104) im Heilungsprozess befindet.

5. Das System (100) nach Anspruch 4, wobei der vorgegebene Bereich ein Bereich zwischen 33 Grad Celsius und 37 Grad Celsius ist.

6. Das System (100) nach einem der vorstehenden Ansprüche, wobei die eine oder die mehreren Sensorvorrichtung(en) einen Feuchtigkeitssensor (420) beinhaltet/beinhalten, und
die vom Computer lesbaren und ausführbaren Befehle die Steuereinheit (140) dazu veranlassen:
basierend auf einem vom Feuchtigkeitssensor (420) gemessenen Feuchtigkeitsgehalt festzustellen, ob im Wundverband (110) eine Sättigung auftritt; und
als Reaktion auf die Feststellung, dass die Sättigung im Wundverband (110) auftritt, eine Warnmeldung über die Netzwerkschnittstellen-Hardware (430) auszugeben.

7. Das System (100) nach einem der vorstehenden Ansprüche, wobei das eine oder die mehreren Speichermodul(e) (144) eine Zeit speichert/speichern, zu der der Wundverband (110) zum ersten Mal verwendet wird, und
die vom Computer lesbaren und ausführbaren Befehle das System (100) dazu veranlassen:
basierend auf dem Zeitpunkt, zu dem der Wundverband (110) zum ersten Mal verwendet wird, festzustellen, ob der Wundverband (110) länger als eine vorgegebene Zeit verwendet wurde; und
als Reaktion auf die Feststellung, dass der Wundverband (110) länger als eine vorgegebene Zeit verwendet wurde, eine Warnmeldung auszugeben.

8. Das System (100) nach Anspruch 7, wobei die vorgegebene Zeit 72 Stunden beträgt.

9. Das System (100) nach einem der vorstehenden Ansprüche, wobei die eine oder die mehreren Sensorvorrichtung(en) einen Biomarkersensor beinhaltet/beinhalten, der so konfiguriert ist, dass ein Anzeichen einer Infektion oder ein Anzeichen für die Heilung der Wunde (104) erkannt wird.

10. Das System (100) nach einem der vorstehenden Ansprüche, wobei der Wundverband (110) eine wundzugewandte Fläche, die so konfiguriert ist, dass sie der Wundfläche zugewandt ist, und eine Außenfläche beinhaltet, und die eine oder die mehreren Sensorvorrichtung(en) auf der wundzugewandten Fläche angebracht sind.

11. Das System (100) nach einem der vorstehenden Ansprüche, femer umfassend ein am Wundverband (110) angebrachtes Heizelement,
wobei:
die eine oder die mehreren Sensorvorrichtung(en) einen für die Messung einer Temperatur konfigurierten Temperatursensor (130) beinhaltet/beinhalten, und
die vom Computer lesbaren und ausführbaren Befehle die Steuereinheit (140) dazu veranlassen, das Heizelement (120) als Reaktion darauf, dass die Temperatur niedriger als eine gewünschte vorgegebene Temperatur ist, zu aktivieren.

12. Das System (100) nach Anspruch 11, ferner umfassend ein Leistungselement (210), das so konfiguriert ist, dass das Heizelement (120) als Reaktion darauf, dass die Temperatur niedriger als eine gewünschte vorgegebene Temperatur ist, zu aktivieren.

13. Das System (100) nach Anspruch 12, wobei das Leistungselement (210) ein photoelektrisches Leistungselement beinhaltet.

14. Das System (100) nach Anspruch 13, wobei die zweite vorgegebene Temperatur 36 Grad Celsius beträgt.

## Revendications

1. Système (100) pour la surveillance d'une plaie (104), comprenant :
un pansement pour plaie (110) conçu pour recouvrir la surface de la plaie (104) ;
un ou plusieurs dispositifs de détection fixés au pansement pour plaie (110), le ou les dispositifs de détection comprenant un pH-mètre (410) ;
un matériel d'interface réseau (430) ; et
un contrôleur (140) comprenant :
un ou plusieurs processeurs (142) ; et
un ou plusieurs modules de mémoire (144) stockant des instructions exécutables et lisibles sur ordinateur qui, lorsqu'elles sont exécutées par un ou plusieurs processeurs (142), amènent le contrôleur (140) à :
recevoir les données mesurées par un ou plusieurs dispositifs de détection ainsi que les temps de mesure ;
déterminer si un niveau de pH mesuré par le pH-mètre (410) augmente avec le temps en fonction des données et des temps de mesure ;
déterminer qu'une infection est susceptible de se produire lorsque le niveau du pH mesuré par le pH-mètre (410) augmente avec le temps ; et
émettre une alerte, via le matériel d'interface réseau (430), en réponse à la détermination de la probabilité de l'infection.

2. Le système (100) selon la revendication 1, dans lequel un ou plusieurs dispositifs de détection comprennent un capteur de température (130), et
les instructions exécutables et lisibles sur ordinateur qui amènent le contrôleur (140) à :
déterminer si une température mesurée par le capteur de température (130) est supérieure à une première température prédéterminée ; et
déterminer qu'une infection est susceptible de se produire en réponse à la détermination que la température est supérieure à la première température prédéterminée.

3. Le système (100) selon la revendication 2, dans lequel la première température prédéterminée est de 38 degrés Celsius.

4. Le système (100) selon l'une quelconque des revendications précédentes, dans lequel un ou plusieurs dispositifs de détection comprennent un capteur de température (130), et
les instructions exécutables et lisibles sur ordinateur qui amènent le contrôleur (140) à :
déterminer si une température mesurée par le capteur de température (130) se trouve dans un intervalle prédéterminé ; et
émettre un message indiquant que la plaie (104) est en cours de cicatrisation en réponse à la détermination que la température se situe dans l'intervalle prédéterminé.

5. Le système (100) selon la revendication 4, où l'intervalle prédéterminé est compris entre 33 degrés Celsius et 37 degrés Celsius.

6. Le système (100) selon l'une quelconque des revendications précédentes, dans lequel un ou plusieurs dispositifs de détection comprennent un capteur d'humidité (420), et
les instructions exécutables et lisibles sur ordinateur qui amènent le contrôleur (140) à :
déterminer si une saturation se produit dans le pansement pour plaie (110) en fonction d'un niveau d'humidité mesuré par le capteur d'humidité (420) ; et
émettre une alerte, via le matériel d'interface réseau (430), en réponse à la détermination que la saturation se produit dans le pansement pour plaie (110).

7. Le système (100) selon l'une quelconque des revendications précédentes, dans lequel un ou plusieurs modules de mémoire (144) enregistrent une heure à laquelle le pansement pour plaie (110) est utilisé pour la première fois, et
les instructions exécutables et lisibles sur ordinateur amènent le système (100) à :
déterminer si le pansement pour plaie (110) a été utilisé pendant une durée supérieure à une durée prédéterminée en fonction du moment où le pansement pour plaie (110) est utilisé pour la première fois ; et
émettre une alerte en réponse à la détermination que le pansement pour plaie (110) a été utilisé pendant une durée supérieure à une durée prédéterminée.

8. Le système (100) selon la revendication 7, dans lequel la durée prédéterminée est de 72 heures.

9. Le système (100) selon l'une quelconque des revendications précédentes, dans lequel un ou plusieurs dispositifs de détection incluent un capteur de biomarqueurs configuré pour détecter une infection ou une cicatrisation de la plaie (104).

10. Le système (100) selon l'une quelconque des revendications précédentes, dans lequel le pansement pour plaie (110) comprend une surface de contact avec la plaie conçue pour recouvrir la surface de la plaie sur la partie externe, et le ou les dispositifs de détection sont fixés sur la surface de contact de la plaie.

11. Le système (100) selon l'une quelconque des revendications précédentes, comprenant en outre un élément chauffant fixé au pansement pour plaie (110),
dans lequel :
le ou les dispositifs de détection incluent un capteur de température (130) configuré pour mesurer une température, et
les instructions exécutables et lisibles sur ordinateur amènent le contrôleur (140) à activer l'élément chauffant (120) en réponse au fait que la température est inférieure à une température prédéterminée souhaitée.

12. Le système (100) selon la revendication 11, comprenant en outre un élément d'alimentation (210) configuré pour activer l'élément chauffant (120) en réponse à une température inférieure à la température prédéterminée souhaitée.

13. Le système (100) selon la revendication 12, dans lequel l'élément d'alimentation (210) comprend un élément d'alimentation photoélectrique.

14. Le système (100) selon la revendication 13, dans lequel la deuxième température prédéterminée est de 36 degrés Celsius.
